# DEMANDE DE BREVET EUROPEEN

(11) **EP 3 748 991 A1**
(43) Date de publication de la demande: **09.12.2020**
(21) Numéro de dépôt: 19305732.0
(22) Date de dépôt: 06.06.2019
(51) Int. Cl.: H04R 25/00, E04B 1/82, A61B 5/12

(54) **CABINE D'AUDIOMÉTRIE AMÉLIORÉE**

(71) Demandeur: Hugon, Bernard, 75014 Paris (FR); Hugonnet, Christian, 75010 Paris (FR)
(72) Inventeur: Hugon, Bernard, 75014 Paris (FR); Hugonnet, Christian, 75010 Paris (FR)
(74) Mandataire: Gauchet, Fabien Roland

(57) **Abrégé**

La cabine d'audiométrie (5), destinée à la prise en charge d'un patient (M) instrumentiste ou mélomane présentant une déficience auditive, comporte un ensemble de parois (51,52,53,54,55,56,57) délimitant un volume acoustique continu formé d'une première zone acoustique (10) « patient » et d'une deuxième zone acoustique (20) « praticien » adjacente à la première zone acoustique, la première zone acoustique étant agencée de sorte à être une zone acoustique diffusante et la deuxième zone acoustique étant agencée de sorte à être une zone acoustique absorbante.

## Description

### DOMAINE TECHNIQUE DE L'INVENTION

L'invention concerne une cabine d'audiométrie destinée à la prise en charge d'un patient mélomane ou instrumentiste présentant une déficience auditive.

### ETAT DE LA TECHNIQUE ANTERIEURE

Actuellement, les aides auditives pour des patients présentant une déficience auditive ont pour vocation d'amplifier la parole de sorte à ce que le patient puisse conserver un lien social en pouvant continuer de communiquer avec autrui. De ce fait, les cabines d'audiométrie des cabinets d'audioprothèses sont conçues dans ce seul but. En effet, la priorité pour un patient déficient auditif, c'est de retrouver la compréhension des mots dans toutes les situations de la vie quotidienne et une communication aisée avec son entourage. Pour répondre à cette demande, les industriels consacrent leur recherche au développement d'algorithmes de reconnaissance de la parole toujours plus performants.

Mais selon des études récentes, 50% des utilisateurs d'aides auditives ne sont pas satisfaits du réglage lorsqu'il s'agit d'écouter de la musique. Dans un exemple célèbre, le compositeur Ludwig van Beethoven avait, en vieillissant, des difficultés pour communiquer avec ses proches mais il souffrait aussi beaucoup de ne plus entendre parfaitement les notes de sa musique. Il a dû renoncer à diriger son orchestre car il ne pouvait plus entendre « sonner » ses partitions. Pour le patient musicien et pour le patient mélomane, la surdité génère une souffrance aussi, voire plus importante que la privation de la parole chez le presbyacousique. En fait, les attentes des musiciens et des mélomanes déficients auditifs nécessitent une prise en charge très différente du patient qui souhaite seulement une amélioration de la compréhension de la parole.

La différence énergétique entre la parole et la musique notamment impose des réglages des aides auditives spécifiques à chacune de ces deux sources sonores. La détermination des réglages spécifiques pour l'écoute de la musique va donc nécessiter des outils originaux qui vont permettre d'abord d'évaluer précisément l'impact de la surdité sur la perception de la musique, ensuite de déterminer, de mettre en oeuvre puis de valider l'efficacité de ces réglages. En particulier, les cabines d'audiométrie actuelles ne permettent pas de remplir ce rôle.

### EXPOSE DE L'INVENTION

Un but de l'invention est de fournir une cabine d'audiométrie permettant la prise en charge d'un patient instrumentiste ou mélomane présentant une déficience auditive de manière optimale.

A cette fin, il est prévu, selon l'invention, une cabine d'audiométrie, destinée à la prise en charge d'un patient instrumentiste ou mélomane présentant une déficience auditive, comportant un ensemble de parois délimitant un volume acoustique continu formé d'une première zone acoustique « patient » et d'une deuxième zone acoustique « praticien » adjacente à la première zone acoustique, la première zone acoustique étant agencée de sorte à être une zone acoustique diffusante et la deuxième zone acoustique est agencée de sorte à être une zone acoustique absorbante.

Avantageusement, mais facultativement, la cabine d'audiométrie selon l'invention présente au moins l'une des caractéristiques techniques suivantes :
- les parois de l'ensemble de parois sont déparallélisées ;
- la première zone acoustique diffusante est délimitée en partie par des parois de l'ensemble de parois comportant des panneaux acoustiques diffuseurs ;
- les panneaux acoustiques diffuseurs sont des panneaux dits de « Schroeder » ;
- la deuxième zone acoustique absorbante est délimitée en partie par des parois de l'ensemble de parois comportant des panneaux acoustiques non réfléchissants phoniques ;
- la cabine d'audiométrie comporte un système de diffusion sonore ;
- la deuxième zone acoustique comporte des premiers émetteurs sonores du système de diffusion sonore ;
- la première zone acoustique comporte des deuxièmes émetteurs sonores du système de diffusion sonore ;
- la cabine d'audiométrie comporte un moniteur de visualisation orienté vers le patient ; et,
- le moniteur de visualisation est positionné dans la deuxième zone acoustique.

### BREVE DESCRIPTION DES FIGURES

D'autres caractéristiques et avantages de l'invention ressortiront à la lecture de la description qui suit d'un mode de réalisation de l'invention. Aux dessins annexés :
- la figure 1 est un plan d'un cabinet d'audioprothèse comportant une cabine d'audiométrie selon l'invention ;
- La figure 2 est un plan de coupe de la cabine d'audiométrie de la figure 1 ;
- La figure 3 est une vue tridimensionnelle d'un panneau de « Schroeder » utilisé dans la cabine d'audiométrie selon l'invention ;
- La figure 4 est une vue de dessus du panneau de la figure 3 ;
- La figure 5 est un plan d'un cabinet d'audioprothèse comportant une variante de réalisation d'une cabine d'audiométrie selon l'invention ; et,
- La figure 6 est un plan de coupe de la cabine d'audiométrie de la figure 5.

Pour plus de clarté, les éléments identiques ou similaires sont repérés par des signes de référence identiques sur l'ensemble des figures.

### DESCRIPTION DETAILLEE D'UN MODE DE REALISATION

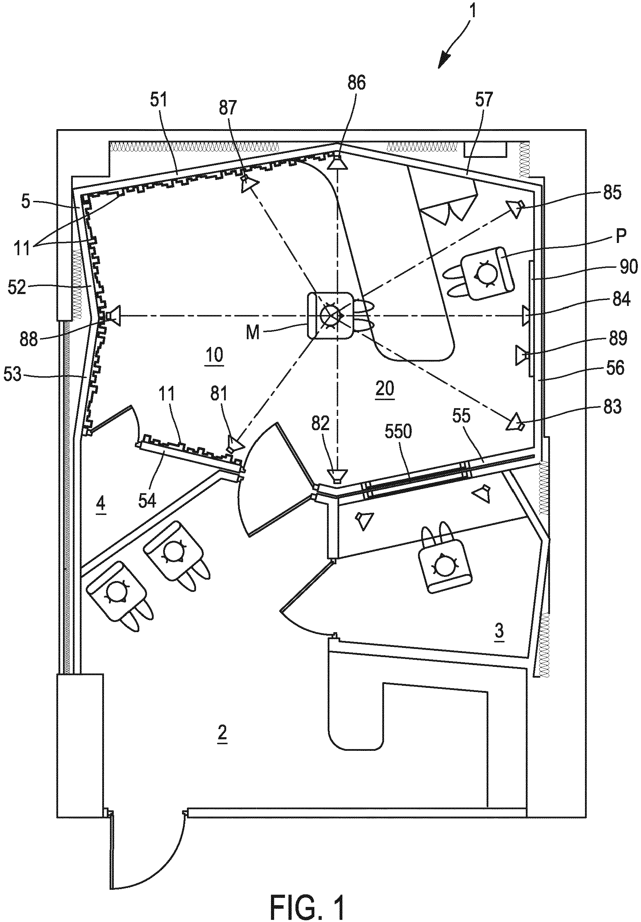

En référence aux figures 1 à 4, nous allons décrire une cabine d'audiométrie 5 selon l'invention. La cabine d'audiométrie 5 selon l'invention est ici intégrée au sein d'un cabinet d'audioprothèse 1. Le cabinet d'audioprothèse 1 comporte en outre, ici, une zone formant accueil-secrétariat 2, un local technique 4 ainsi qu'un laboratoire/régie 3.

La cabine d'audiométrie 5 selon l'invention comporte un ensemble de parois 50 à 59 qui délimite un volume acoustique continu 10,20. L'ensemble de parois comprend une première série de parois latérales 51 à 57 formant les murs de la cabine d'audiométrie 5 selon l'invention, une paroi formant plancher 58 et une deuxième série de parois formant faux-plafond 50 et 59. Les parois 50 à 59 sont déparallélisées : deux parois de l'ensemble de parois qui s'étendent en regard l'une de l'autre ne sont pas parallèles l'une par rapport à l'autre. Un tel agencement des parois 50 à 59 de l'ensemble de parois de la cabine d'audiométrie 5 selon l'invention permet de neutraliser les phénomènes d'ondes sonores stationnaires lors de l'émission d'un son dans le volume acoustique continu de la cabine d'audiométrie 5 selon l'invention.

Au niveau d'un plafond existant 6 du cabinet d'audioprothèse 1, le plafond 6 est doublé par un complexe isolant comportant ici une paroi de plâtre phonique 61 ou assimilée qui est désolidarisée phoniquement du plafond 6 par une ossature autoportante 63 qui peut être réalisée à partir de profilés Stil Prim ® de la société Placoplatre par exemple. De plus, le complexe comporte un matelas 62 de laine minérale ou assimilé déposé sur la paroi de plâtre phonique 61 : par exemple, le matelas 52 présente une épaisseur de l'ordre de 100mm et une densité de l'ordre de 17kg.m⁻³.

Le volume acoustique continu ainsi délimité est formé d'une première zone acoustique 10 « patient » dans laquelle se place un patient M musicien ou mélomane présentant une déficience auditive et d'une deuxième zone acoustique 20 « praticien », adjacente à la première zone acoustique, et dans laquelle se place un praticien P, en particulier un audioprothésiste ou assimilé.

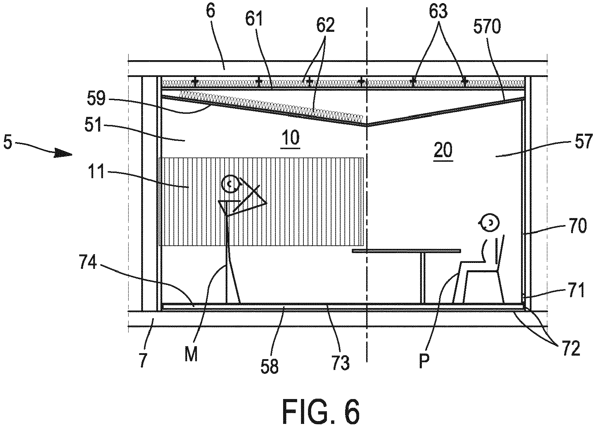

La paroi formant plancher 58 est commune aux première 10 et deuxième 20 zones acoustiques de la cabine d'audiométrie 5 selon l'invention. La paroi 58 comporte une couche de matériau résilient 72 de l'ordre, ici, d'une vingtaine de millimètres d'épaisseur posée sur un sol existant 7 du cabinet d'audioprothèse 1, puis d'une chape de béton 74, ici, de l'ordre de 70mm d'épaisseur. En variante, comme illustré à la figure 6, la couche de matériau résilient 72 remonte latéralement sur une périphérie de la chape de béton 74. La remontée de la couche de matériau résilient 72 se prolonge au-dessus par une plinthe 71. Un parquet 73 est posé sur la chape de béton 74.

La première zone acoustique 10 est délimitée en partie par un premier sous ensemble de parois 51,52,53,54,58,59 de l'ensemble de parois de la cabine d'audiométrie 5 selon l'invention. Les parois latérales 51,52,53 formant mur comportent chacune une paroi de plâtre phonique montée sur une ossature simple. Cette dernière est positionnée à distance des murs existants du cabinet d'audioprothèse 1. Une épaisseur (de l'ordre de 45mm par exemple) de laine minérale est prévue dans l'ossature. Au niveau d'une jonction des parois latérales 51,52,53 avec le plafond 6 et un plancher 7 existants du cabinet d'audioprothèse, un bloc de matériau résilient est interposé. Ce bloc de matériau résilient présente une épaisseur de l'ordre de 10mm.

La paroi latérale 54 sépare la cabine d'audiométrie 5 selon l'invention du local technique 4 et comporte des première et deuxième parois de plâtre phonique fixées de part et d'autre d'une ossature simple. Par exemple les première et deuxième parois de plâtre phonique comportent chacune deux plaques de plâtre phonique superposées en pose croisée. Une épaisseur (de l'ordre de 40mm par exemple) est interposée dans l'ossature entre les deux parois de plâtre phonique. De nouveau, au niveau d'une jonction de la paroi latérale 54 avec le plafond 6 et le plancher 7, un bloc de matériau résilient est interposé. Ce bloc de matériau résilient présente une épaisseur de l'ordre de 10mm.

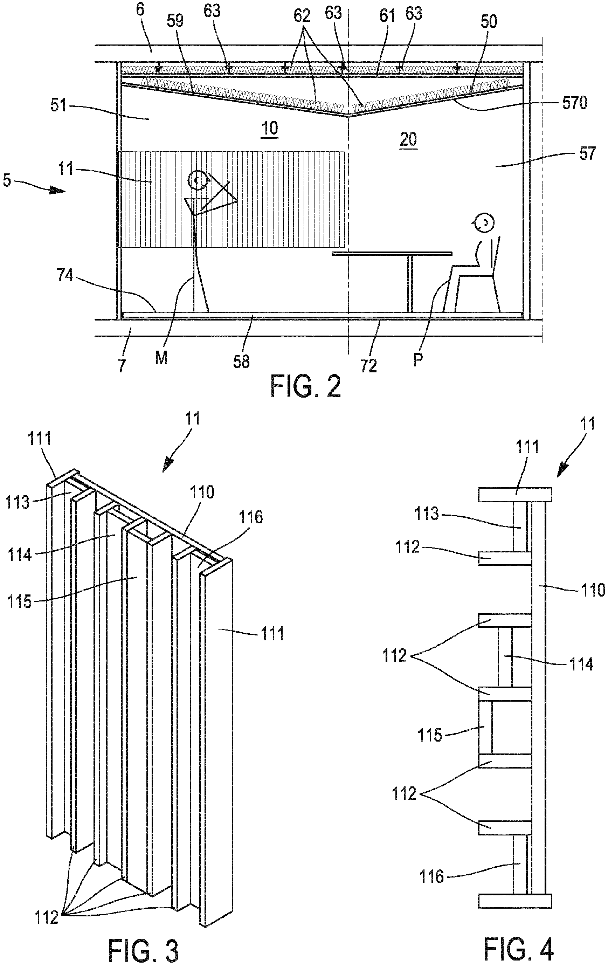

La paroi 59 formant faux-plafond comporte une paroi en bois sur un support autoportant sur laquelle est positionné un matelas 62 de laine minérale. La paroi 59 est inclinée par rapport à un plan horizontal de sorte à ne pas être parallèle avec la paroi 58 formant plancher qui lui est en regard. En particulier, la paroi 59 est inclinée vers l'intérieur de la cabine d'audiométrie 5 selon l'invention comme cela est illustré à la figure 2.

Les parois latérales 51,52,53,54 comportent des panneaux diffuseurs 11 qui permettent de diffuser par réflexion un signal sonore émis dans le volume acoustique continu 10,20. En particulier, le son d'un instrument de musique joué par le patient M dans la première zone acoustique 10. En particulier les panneaux diffuseurs 11 sont des panneaux diffuseurs acoustiques de Schroeder permettant à une onde acoustique percutant un tel diffuseur acoustique de réfléchir l'onde sonore dans plusieurs angles en diminuant l'ensemble de l'intensité sonore sans changer le timbre. Cela permet d'obtenir une diffusion très homogène de l'onde sonore dans le volume acoustique continu et en particulier dans la première zone acoustique. Les panneaux diffuseurs de Schroeder sont connus en soi. Ici le panneau diffuseur 11 de Schroeder comporte un panneau de base 110 bordé latéralement par deux parois verticales d'extrémité 111 qui s'étendent en saillie d'une face du panneau de base 110 qui est orientée vers la première zone acoustique 10, lors d'un montage. Entre les deux parois verticales d'extrémité 111, le panneau diffuseur 11 de Schroeder comprend une série de parois verticales intermédiaires 112 s'étendant en saillie depuis la face du panneau de base 110 orientée vers la première zone acoustique 10, lors d'un montage. D'autre part, les parois verticales intermédiaires 112 sont réparties uniformément entre les deux parois verticales d'extrémité 111. De plus, les parois verticales intermédiaires 112 sont ici au nombre de cinq. Entre deux parois verticales intermédiaires 112 et d'extrémité 111 adjacentes délimitant une gorge verticale, le panneau diffuseur 11 de Schroeder comporte une paroi de fond 113,114,115,116 positionnée à une profondeur prédéterminée dans la gorge verticale considérée. Cette profondeur prédéterminée est différente entre deux gorges verticales adjacentes. De plus le panneau de base 110 sert de paroi de fond pour certaines gorges verticales. Un tel panneau diffuseur 11 de Schroeder est illustré en figures 3 et 4.

Un tel agencement de la première zone acoustique 10 permet à celle-ci d'être une zone acoustique diffusante. Cette zone diffusante est adaptée au jeu instrumental du patient M. Elle est bénéfique au timbre de l'instrument joué ainsi qu'à l'écoute individuelle du patient M. Ainsi, le patient M dispose d'un contrôle optimal de la sonorité de son instrument grâce à la multiplication des réflexions des ondes sonores générées par l'instrument, réflexions sonores apportées par les panneaux diffuseurs 11.

La deuxième zone acoustique 20 est délimitée en partie par un deuxième sous ensemble de parois 55,56,57,58,50 de l'ensemble de parois de la cabine d'audiométrie 5 selon l'invention. Les parois latérales 56,57 formant mur comportent chacune une paroi de plâtre phonique montée sur une ossature simple. Cette dernière est positionnée à distance des murs existants du cabinet d'audioprothèse 1. Une épaisseur (de l'ordre de 45mm par exemple) de laine minérale est prévue dans l'ossature. Au niveau d'une jonction des parois latérales 56,57 avec le plafond 6 et un plancher 7 existants du cabinet d'audioprothèse, un bloc de matériau résilient est interposé. Ce bloc de matériau résilient présente une épaisseur de l'ordre de 10mm.

La paroi latérale 55 sépare la cabine d'audiométrie 5 selon l'invention du laboratoire/régie 3 et comporte des première et deuxième parois de plâtre phonique fixées de part et d'autre d'une ossature double formée de deux ossatures simples superposées. Par exemple, les première et deuxième parois de plâtre phonique comportent chacune deux plaques de plâtre phonique superposées en pose croisée. Une épaisseur (de l'ordre de 40mm par exemple) est interposée dans chacune des ossatures simples de l'ossature double entre les deux parois de plâtre phonique. De nouveau, au niveau d'une jonction de la paroi latérale 55 avec le plafond 6 et le plancher 7, un bloc de matériau résilient est interposé. Ce bloc de matériau résilient présente une épaisseur de l'ordre de 10mm. D'autre part, la paroi latérale 55 comporte une ouverture vitrée 550. L'ouverture vitrée 550 est par exemple formée de deux châssis vitrés espacés l'un de l'autre par une cale d'espacement absorbante phoniquement. Les deux châssis vitrés sont positionnés de sorte que les deux vitrages qu'il comprennent ne soient pas parallèles l'un par rapport à l'autre.

La paroi 50 formant faux-plafond comporte un panneau absorbant phoniquement 570 sur un support autoportant sur lequel est positionné un matelas 62 de laine minérale. La paroi 50 est inclinée par rapport à un plan horizontal de sorte à ne pas être parallèle avec la paroi 58 formant plancher qui lui est en regard. En particulier, la paroi 50 est inclinée vers l'intérieur de la cabine d'audiométrie 5 selon l'invention comme cela est illustré à la figure 2.

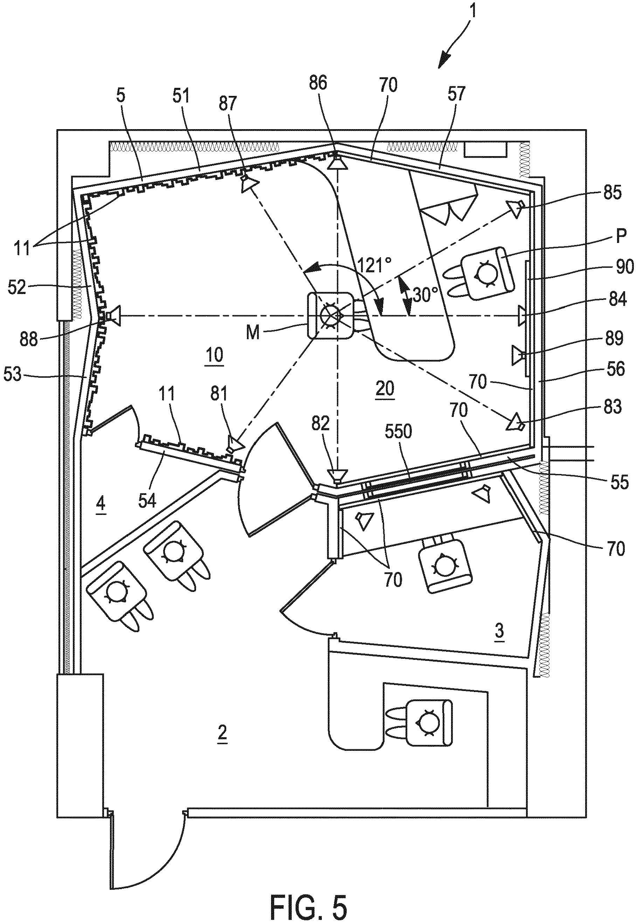

En variante de réalisation, l'effet absorbant recherché des parois latérales 55,56,57 de la deuxième zone acoustique 20 est renforcé en les doublant par l'intérieur de la cabine d'audiométrie 5 selon l'invention d'un panneau absorbant phoniquement 70 comme cela est illustré en figures 5 et 6.

Un tel agencement de la deuxième zone acoustique 20 permet à celle-ci d'être une zone acoustique absorbante. En effet, les parois de plâtre acoustiques agissent comme des panneaux acoustiques non réfléchissants phoniques. Il en est de même des panneaux absorbants phoniquement qui sont non réfléchissants phoniques. Cette zone acoustique absorbante est dédiée à la reproduction sur un système de diffusion sonore décrit ci-après. Elle permet des écoutes précises et une évaluation optimale de la déficience auditive du patient M. Le système de diffusion sonore fournit au patient M un son direct sans réflexions déformantes et permet l'optimisation fine du réglage des aides auditives portées par le patient M.

D'autre part, la cabine d'audiométrie 5 selon l'invention comporte un système de diffusion sonore 81 à 89. Le système de diffusion sonore comprend des premiers émetteurs sonores 83,84,85 qui sont positionnés dans la deuxième zone acoustique 20. Les émetteurs sonores 83,84,85 sont orientés vers le patient M quand celui-ci est assis au niveau d'une limite entre les première 10 et deuxième 20 zones acoustiques, comme illustré sur les figures 1 et 5. L'émetteur sonore 84 est, ici face au patient M alors que les émetteurs sonores 83 et 85 sont décalés de 30° de part et d'autre de l'émetteur sonore 84. D'autre part, le système de diffusion sonore comporte des deuxièmes émetteurs sonores 81,87,88 positionnés dans la première zone acoustique 10. De nouveau, les émetteurs sonores 81,87,88 sont orientés vers le patient M assis à la limite des première 10 et deuxième 20 zones acoustiques. Ainsi, les émetteurs sonores 81,87,88 sont en arrière du patient M, les émetteurs sonores 81,87 étant décalés de 121° de part et d'autre de l'émetteur sonore 84 et l'émetteur sonore 88 faisant face à l'émetteur sonore 84. Enfin, ici, le système de diffusion sonore comporte des troisièmes émetteurs sonores 82,86 positionnés à la limite des première 10 et deuxième 20 zones acoustiques de la cabine d'audiométrie 5 selon l'invention. De nouveau, les émetteurs sonores 82,86 sont orientés vers la patient M assis à la limite des première 10 et deuxième 20 zones acoustiques. Ainsi, les émetteurs sonores 82, 86 sont à la gauche et à la droite du patient M, décalés de 90° de part et d'autre de l'émetteur sonore 84. D'autre part, le système de diffusion sonore comporte, en outre, un émetteur sonore 89 qui est un caisson de basse ou de grave positionné dans la deuxième zone acoustique 20 et dont le rôle est de diffuser le spectre basse fréquence. Ainsi, tel qu'il vient d'être décrit, le système de diffusion 81 à 89 forme un système sonore 8.1.

Le système de diffusion sonore 81 à 89 qui vient d'être décrit est couplé à des moyens de mesures audiologiques (non représentés). Il permet ainsi de tester l'audition musicale du patient M dans toutes les situations possibles tant pour un patient M instrumentiste en situation de jeu que pour un patient M mélomane.

Pour le patient M instrumentiste, cela permet de régler les aides auditives en situation de jeu instrumental en reconstituant la sonorité de salles de concert connues. En jeu en soliste, le son de l'instrument du patient M instrumentiste est capté puis rediffusé dans le système de diffusion sonore après avoir été traité avec des réverbérations sophistiquées qui vont reproduire la couleur d'une salle de concert donnée. A ce moment-là, le patient M instrumentiste a l'illusion de jouer dans cette salle. Il peut ainsi évaluer la qualité du réglage de ses aides auditives. En immersion dans un orchestre de chambre (jeu en formation musique de chambre), l'utilisation du système de diffusion sonore permet de diffuser les autres pupitres de la formation chambriste comme un quatuor, un quintet... Le patient M instrumentiste joue et entend tous les autres pupitres de la formation. En immersion dans un orchestre symphonique (jeu en formation d'ensemble orchestral), l'utilisation du système de diffusion sonore permet de placer le patient M instrumentiste à sa place au sein de l'orchestre.

Pour le patient M mélomane, cela permet de tester les aides auditives pour une écoute idéale de la musique. L'évaluation de la dégradation de la qualité d'écoute musicale du patient M se fait sur la base de tests dont les conditions de passation doivent être fiables et reproductibles. Le système de diffusion sonore permet notamment de réaliser un test de reconnaissance de timbre par passation dans toutes les configurations pour mesurer le plus précisément possible la qualité sonore avec les aides auditives. Par exemple, l'utilisation du système de diffusion sonore en mode stéréophonique permet de réaliser une écoute en stéréophonie dans un environnement très amorti, le patient M mélomane capte uniquement le son direct. Ainsi il peut se concentrer sur la qualité des timbres et sur le test lui-même. Cette configuration va permettre une fiabilité et une reproductibilité totale des tests proposés. D'autre part, l'utilisation du système de diffusion sonore en mode 5.1 ou 7.1 permet la diffusion d'enregistrements (DVD ou Blue Ray) en 5.1 ou en 7.1 afin de fournir au patient M mélomane une écoute de leur propre musique et de tester l'efficacité de leurs aides auditives avec des enregistrements connus.

La cabine d'audiométrie 5 selon l'invention comporte aussi un moniteur de visualisation 90 positionné ici dans la deuxième zone acoustique 20. Il est ici accroché sur la paroi latérale 56. Il est orienté vers le patient. L'utilisation d'un tel moniteur permet, pour le patient M, simultanément à l'écoute d'un morceau, de suivre la partition correspondante ou de visualiser les aspects harmoniques du même morceau. Cela permet de valider le réglage des aides auditives ou de corriger d'éventuels défauts. Pour cela, un enregistrement est diffusé dans le système de diffusion sonore et la partition défile simultanément sur le moniteur de visualisation 90. Le but est de vérifier la qualité sonore de l'aide auditive. En effet, si un défaut de sonorité se manifeste au passage d'une note (grésillement, sifflement...) son identification sur la partition permet d'intervenir avec précision dans le réglage et d'avoir une action correctrice sur le ou les paramètres concernés de l'aide auditive. D'autre part, l'utilisation du moniteur de visualisation 90 permet de rééduquer l'écoute analytique et, donc, de retrouver le chemin du plaisir musical. A cette fin, un enregistrement est diffusé par le système de diffusion sonore et le moniteur de visualisation affiche les notes, les harmonies et la tonalité en temps réel. Cette situation d'écoute-visualisation va permettre une rééducation de l'oreille appareillée du patient M. Le but est de reconstruire cette écoute analytique propre au patient M qu'il soit instrumentiste ou mélomane lorsqu'il est en présence de musique et l'aider à retrouver le chemin du plaisir de l'écoute.

L'utilisation de la cabine d'audiométrie 5 selon l'invention qui vient d'être décrite permet donc à un patient M d'écouter correctement de la musique malgré sa déficience auditive en ciblant tous les aspects spécifiques du langage musical (note, rythme, nuance, harmonie, tonalité, timbre, couleur). La cabine d'audiométrie 5 selon l'invention va permettre d'évaluer l'impact de la surdité du patient M sur la perception de la musique, de visualiser en temps réel le signal sonore devant le ou les tympans du patient M, d'optimiser les réglages des aides auditives *in vivo.* Il est possible aussi d'explorer les aspects psychoacoustiques afin de mieux comprendre l'audition du patient M : évaluation du pouvoir séparateur en intensité et en fréquence qui indique la précision de l'oreille dans ces domaines, mesurer les performances en localisation spatiale indispensable pour écouter un orchestre, évaluer la reconnaissance des timbres des différents instruments pour le plaisir de l'écoute, la reconnaissance de la tonalité pour apprécier la pensée du compositeur, déterminer la qualité perçue pour savoir comment le patient M vit ses aides auditives, identifier des émotions du patient M pendant l'écoute de musique.

Bien entendu, il est possible d'apporter à l'invention de nombreuses modifications sans pour autant sortir du cadre de celle-ci.

## Revendications

1. Cabine d'audiométrie (5), destinée à la prise en charge d'un patient (M) instrumentiste ou mélomane présentant une déficience auditive, comportant un ensemble de parois (50,51,52,53,54,55,56,57,58,59) délimitant un volume acoustique continu formé d'une première zone acoustique (10) « patient » et d'une deuxième zone acoustique (20) « praticien » adjacente à la première zone acoustique, **caractérisée en ce que** la première zone acoustique est agencée de sorte à être une zone acoustique diffusante et la deuxième zone acoustique est agencée de sorte à être une zone acoustique absorbante.

2. Cabine d'audiométrie selon la revendication 1, **caractérisée en ce que** les parois de l'ensemble de parois sont déparallélisées.

3. Cabine d'audiométrie selon la revendication 1 ou la revendication 2, **caractérisée en ce que** la première zone acoustique diffusante est délimitée en partie par des parois (51,52,53,54,58,59) de l'ensemble de parois comportant des panneaux acoustiques diffuseurs (11).

4. Cabine d'audiométrie selon la revendication 3, **caractérisée en ce que** les panneaux acoustiques diffuseurs sont des panneaux dits de « Schroeder ».

5. Cabine d'audiométrie selon l'une des revendications 1 à 4, **caractérisée en ce que** la deuxième zone acoustique absorbante est délimitée en partie par des parois (50,55,56,57,58) de l'ensemble de parois comportant des panneaux acoustiques non réfléchissants phoniques.

6. Cabine d'audiométrie selon l'une des revendications 1 à 5, **caractérisée en ce qu'**elle comporte un système de diffusion sonore (81,82,83,84,85,86,87,88,89).

7. Cabine d'audiométrie selon la revendication 6, **caractérisée en ce que** la deuxième zone acoustique comporte des premiers émetteurs sonores (83,84,85,89) du système de diffusion sonore.

8. Cabine d'audiométrie selon la revendication 6 ou la revendication 7, **caractérisée en ce que** la première zone acoustique comporte des deuxièmes émetteurs sonores (81,87,88) du système de diffusion sonore.

9. Cabine d'audiométrie selon l'une des revendications 1 à 8, **caractérisée en ce qu'**elle comporte un moniteur de visualisation (90) orienté vers le patient.

10. Cabine d'audiométrie selon la revendication 9, **caractérisée en ce que** le moniteur de visualisation est positionné dans la deuxième zone acoustique.
